# EUROPEAN PATENT APPLICATION

(11) **EP 4 785 968 A1**
(43) Date of publication of application: **05.08.2026**
(21) Application number: 24884386.4
(22) Date of filing: 10.10.2024
(51) Int. Cl.: A61L 27/38

(54) **TISSUE-ENGINEERED ARTIFICIAL BLOOD VESSEL, PREPARATION METHOD THEREFOR, AND USE THEREOF**

(30) Priority: 03.11.2023 CN 202311456027
(71) Applicant: Roumai Medical (Shenzhen) Co., Ltd, Shenzhen, Guangdong 518055 (CN); Roumai Medical (Switzerland) SA, Lausanne 1000-1018 (CH)
(72) Inventor: CHENG, Shiyu, Shenzhen, Guangdong 518055 (CN); WANG, Junbiao, Shenzhen, Guangdong 518055 (CN)
(74) Representative: Heilein, Ernst-Peter
(86) International application number: PCT/CN2024/123938
(87) International publication number: WO 2025/092382

(57) **Abstract**

A tissue-engineered artificial blood vessel, a preparation method therefor, and use thereof. The preparation method comprises: inoculating vascular endothelial cells and smooth muscle cells in an inner layer of a tubular cell scaffold and culturing in a mixed manner for 7-13 days; changing to a pro-secretory medium and meanwhile applying a perfusion pulse pressure in the tubular cell scaffold, culturing for another 8-13 days, inoculating vascular fibroblasts in an outer layer of the tubular cell scaffold and culturing the cells, and changing to a mixed medium and co-culturing for 8-13 days; and replacing the mixed medium with a pro-secretory medium and co-culturing for 10-16 days and then decellularizing in situ to obtain the tissue-engineered artificial blood vessel. The preparation method can promote extracellular matrix secretion, enabling the prepared artificial blood vessel to have better mechanical strength and elasticity, and shorten culture time.

## Description

### TECHNICAL FIELD

The present disclosure relates to the field of tissue engineering technology, and in particular to a tissue-engineered artificial blood vessel and a preparation method and use thereof.

### BACKGROUND

Human blood vessels are mainly composed of, from inside to outside, endothelial cells, smooth muscle cells, and fibroblasts, as well as the extracellular matrix secreted by these three types of cells. These cells and their extracellular matrix play different roles in maintaining vascular structure and patency. Vascular diseases, which are currently very common and have a high mortality rate, are typically caused by vascular stenosis or occlusion, leading to reduced blood flow and nutrient deficiency, thereby damaging tissues or organs. Using autologous blood vessels from patients for vascular graft surgery is a conventional treatment for vascular diseases. However, harvesting autologous blood vessels from patients faces challenges such as limited available vessel length, mismatched vessel caliber, and difficulty in finding vessels meeting requirements. Therefore, artificial blood vessels represent a highly promising solution.

Currently, technologies for preparing artificial blood vessels using materials such as polyethylene terephthalate (PET), expanded polytetrafluoroethylene (ePTFE), PCL, PLCL, PU, PGS, PLA, PDO, and collagen still face difficulties in preparing small-caliber blood vessels with inner diameters less than 6 mm, lack of biological activity in prepared vessels, slow vascular regeneration, and poor long-term patency. This is because the lumen of artificial blood vessels prepared from these materials lacks a physiologically functional endothelial lining. Therefore, researchers have attempted to construct endothelialized artificial blood vessels by seeding endothelial cells in vitro through tissue engineering. However, due to significant differences between in vitro culture conditions and the in vivo environment, and the lack of support from smooth muscle cells for the endothelial cells, such artificial blood vessels are prone to endothelial detachment, leading to thrombosis.

Furthermore, the culture methods for tissue-engineered blood vessels in related technologies mostly involve culturing a single cell type in a single cell culture medium in containers such as in vitro bioreactors to form tissues. However, the cell types and culture methods do not conform to the actual conditions of blood vessels in the human body. Such technologies also face problems of poor ability of cells to secrete extracellular matrix components such as structural and supportive proteins, resulting in poor vascular tissue remodeling capability and difficulty in forming artificial blood vessels with high elasticity, good mechanical properties, and biocompatibility. Meanwhile, obtaining artificial blood vessels through co-culture of three cell types also faces the challenge of allowing different cells to grow in the same environment while obtaining their respective required cytokines. Moreover, current tissue engineering culture methods for artificial blood vessels in related technologies require more than 8 weeks, leading to high costs. Long-term cell culture results in bacterial and fungal infections, significantly increasing the risk of microbial contamination.

Therefore, there is an urgent need for a tissue-engineered artificial blood vessel having high elasticity, good mechanical properties and biocompatibility, excellent long-term patency, and a preparation method for the tissue-engineered artificial blood vessel, through which a small-caliber blood vessels with an inner diameter of less than 6 mm can be prepared.

### SUMMARY

The present disclosure aims to solve at least one of the technical problems existing in the existing technology. To this end, the present disclosure provides a preparation method for a tissue-engineered artificial blood vessel. The preparation method of the present disclosure can prepare a small-caliber artificial blood vessel with an inner diameter of less than 6 mm, and promotes substantial secretion of extracellular matrix components such as structural proteins and supportive proteins by cells during the preparation and culture process. This enables the prepared tissue-engineered artificial blood vessel to have high elasticity, good mechanical properties and biocompatibility, and excellent long-term patency, allowing it to substitute for natural blood vessels of humans or animals. In addition, the preparation method of the present disclosure shortens the culture time, reduces costs, and mitigates the risk of contamination.

The present disclosure further provides a tissue-engineered artificial blood vessel.

The present disclosure further provides use of the above preparation method and tissue-engineered artificial blood vessel.

In a first aspect, the present disclosure provides a preparation method for a tissue-engineered artificial blood vessel, including the following steps:
S1: seeding vascular endothelial cells and smooth muscle cells on an inner layer of a tubular cellular scaffold, and performing mixed culture for 7 to 13 days;
S2: changing to a secretion-promoting culture medium, simultaneously applying perfusion pulsatile pressure within the tubular cellular scaffold, continuing culture for 8 to 13 days, then seeding vascular fibroblasts on an outer layer of the tubular cellular scaffold for culture, and changing to a mixed culture medium for co-culture for 8 to 13 days; and
S3: replacing the mixed culture medium with the secretion-promoting culture medium, co-culturing for 10 to 16 days, followed by in-situ decellularization treatment to obtain the tissue-engineered artificial blood vessel;
where the secretion-promoting culture medium includes cytokines, amino acids, vitamins, and serum or serum substitute.

According to specific embodiments of the present disclosure, the preparation method for a tissue-engineered artificial blood vessel of the present disclosure can prepare small-caliber artificial blood vessels with an inner diameter of less than 6 mm. During the culture process, it can promote interactions among various cells and facilitate substantial secretion of extracellular matrix components by cells. This enables the prepared tissue-engineered artificial blood vessel to have high elasticity, good mechanical properties and biocompatibility. The artificial blood vessel prepared by the present disclosure has high contents of structural and supportive proteins, can maintain favorable elasticity and mechanical properties, and has excellent long-term patency. In addition, the preparation method of the present disclosure employs simultaneous culture of three types of cells, shortening the culture time, reducing costs, and mitigating the risk of contamination.

In some embodiments of the present disclosure, the endothelial cells are derived from an aorta and an umbilical artery and vein.

In some preferred embodiments of the present disclosure, the endothelial cells are derived from the umbilical artery.

In some embodiments of the present disclosure, the smooth muscle cells are derived from the aorta and the umbilical artery and vein.

In some preferred embodiments of the present disclosure, the smooth muscle cells are derived from the umbilical artery.

In some embodiments of the present disclosure, the fibroblasts are derived from the aorta and the umbilical artery and vein.

In some preferred embodiments of the present disclosure, the fibroblasts are derived from the umbilical artery.

In some embodiments of the present disclosure, the endothelial cells have a seeding density of 100,000-1,500,000 cells/cm.

In some preferred embodiments of the present disclosure, the endothelial cells have a seeding density of 200,000-1,000,000 cells/cm.

In some embodiments of the present disclosure, the smooth muscle cells have a seeding density of 1,000,000-7,000,000 cells/cm.

In some preferred embodiments of the present disclosure, the smooth muscle cells have a seeding density of 2,000,000-6,000,000 cells/cm.

In some more preferred embodiments of the present disclosure, the smooth muscle cells have a seeding density of 2,000,000-5,000,000 cells/cm.

In some embodiments of the present disclosure, the fibroblasts have a seeding density of 500,000-7,000,000 cells/cm.

In some preferred embodiments of the present disclosure, the fibroblasts have a seeding density of 1,000,000-4,000,000 cells/cm.

In some more preferred embodiments of the present disclosure, the fibroblasts have a seeding density of 1,000,000-3,000,000 cells/cm.

In some embodiments of the present disclosure, the tubular cellular scaffold is made of a biodegradable polymer material.

In some embodiments of the present disclosure, the tubular cellular scaffold includes at least one of polylactic acid (PLA), polyglycolic acid (PGA), polycaprolactone (PCL), poly(lactic-co-glycolic acid) (PLGA), polyhydroxyalkanoates (PHA), and collagen.

In some preferred embodiments of the present disclosure, the tubular cellular scaffold includes polyglycolic acid (PGA).

In some embodiments of the present disclosure, the tubular cellular scaffold has a wall thickness of 0.1-2.5 mm.

In some preferred embodiments of the present disclosure, the tubular cellular scaffold has a wall thickness of 0.5-1.5 mm.

In some more preferred embodiments of the present disclosure, the tubular cellular scaffold has a wall thickness of 0.8-1 mm.

In some embodiments of the present disclosure, applying perfusion pulsatile pressure in step S2 specifically includes: initially applying 1-10 mmHg, then increasing by 3-20 mmHg daily.

In some preferred embodiments of the present disclosure, applying perfusion pulsatile pressure in step S2 specifically includes: initially applying 1-10 mmHg, then increasing by 5-15 mmHg daily.

In some preferred embodiments of the present disclosure, applying perfusion pulsatile pressure in step S2 specifically includes: initially applying 1-10 mmHg, then increasing by 5-15 mmHg daily, with the applied perfusion pulsatile pressure ranging between 1-300 mmHg.

In the culture and preparation process of the artificial blood vessel of the present disclosure, pulsatile pressure is applied and gradually increased within an appropriate range. Applying pulsatile pressure helps promote cell growth on the artificial blood vessel and secretion of extracellular matrix, facilitates the formation of vascular tissue, shortens the culture time to a certain extent, and effectively improves properties such as mechanical strength and elasticity of the artificial blood vessel.

In some embodiments of the present disclosure, the culture medium for the mixed culture in step S1 is a mixture of a vascular endothelial cell culture medium and a smooth muscle cell culture medium in a volume ratio of 1: 2-10.

In some preferred embodiments of the present disclosure, the culture medium for the mixed culture in step S1 is a mixture of a vascular endothelial cell culture medium and a smooth muscle cell culture medium in a volume ratio of 1: 4.

In some embodiments of the present disclosure, the culture medium for the mixed culture in step S1 contains serum or serum substitute HPL at a mass concentration of 10%-25%.

In some preferred embodiments of the present disclosure, the culture medium for the mixed culture in step S1 contains serum at a mass concentration of 15%-25% or serum substitute at a mass concentration of 10%-20%.

In some more preferred embodiments of the present disclosure, the culture medium for the mixed culture in step S1 contains serum at a mass concentration of 18%-22% or serum substitute at a mass concentration of 13%-17%.

In some embodiments of the present disclosure, the mixed culture medium in step S2 is a mixture of a vascular endothelial cell culture medium, a smooth muscle cell culture medium, and a fibroblast culture medium in a volume ratio of 1: 4-10: 2-5.

In some preferred embodiments of the present disclosure, the mixed culture medium in step S2 is a mixture of an endothelial cell culture medium, a smooth muscle cell culture medium, and a fibroblast culture medium in a volume ratio of 1: 6: 3.

In some embodiments of the present disclosure, the mixed culture medium in step S2 contains serum or serum substitute at a mass concentration of 5-15%.

In some preferred embodiments of the present disclosure, the mixed culture medium in step S2 contains serum or serum substitute at a mass concentration of 8-12%.

In some embodiments of the present disclosure, the serum substitute is Human Platelet Lysate (HPL).

The present disclosure adopts a scheme of simultaneously culturing several types of vascular cells. By screening and optimizing the ratios of different culture media, a suitable multi-cell co-culture medium formulation is obtained, thereby achieving optimization of the growth rate of each cell type and enhancement of the interaction between cells, while also shortening the culture time to a certain extent.

In some embodiments of the present disclosure, the secretion-promoting culture medium in step S2 includes: cytokines, amino acids, vitamins, and 8%-12% serum or serum substitute.

In some embodiments of the present disclosure, the cytokines include at least one of VEGF, PDGF, IGF, HGF, TGF-β1, TGF-β2, TGF-β3, and BMP.

In some embodiments of the present disclosure, the cytokines include at least one of VEGF, PDGF, IGF, HGF, TGF-β1, TGF-β2, TGF-β3, and BMP, with cytokine concentrations independently being 1-20 ng/mL;
In some preferred embodiments of the present disclosure, the cytokines include 1-5 cytokines selected from VEGF, PDGF, IGF, HGF, TGF-β1, TGF-β2, TGF-β3, and BMP, with cytokine concentrations independently being 1-20 ng/mL.

In some more preferred embodiments of the present disclosure, the cytokines include 1-5 cytokines selected from VEGF, PDGF, IGF, HGF, TGF-β1, TGF-β2, TGF-β3, and BMP, with all cytokine concentrations being 5-15 ng/mL.

In some more preferred embodiments of the present disclosure, the cytokines include VEGF, TGF-β1, and TGF-β2, with cytokine concentrations independently being 5-15 ng/mL.

In some more preferred embodiments of the present disclosure, the cytokines are VEGF, TGF-β1, and TGF-β2, with cytokine concentrations independently being 5-15 ng/mL.

In some embodiments of the present disclosure, the vitamins include vitamin B and vitamin C.

In some preferred embodiments of the present disclosure, the vitamins include vitamin B and vitamin C, with concentrations independently being 30-100 mg/mL.

In some embodiments of the present disclosure, the amino acids include glycine, alanine, glutamic acid, and proline.

In some preferred embodiments of the present disclosure, the amino acids include glycine, alanine, glutamic acid, and proline, with concentrations independently being 20-60 mg/mL.

In some embodiments of the present disclosure, the serum substitute is Human Platelet Lysate (HPL).

In some embodiments of the present disclosure, the secretion-promoting culture medium in step S3 includes: cytokines, amino acids, vitamins, and 3%-7% serum or serum substitute.

In some embodiments of the present disclosure, the cytokines include at least one of bFGF, IGF, HGF, TGF-β1, TGF-β2, TGF-β3, and BMP.

In some embodiments of the present disclosure, the cytokines include at least one of bFGF, IGF, HGF, TGF-β1, TGF-β2, TGF-β3, and BMP, with cytokine concentrations independently being 1-20 ng/mL;
In some preferred embodiments of the present disclosure, the cytokines include 1-5 cytokines selected from bFGF, IGF, HGF, TGF-β1, TGF-β2, TGF-β3, and BMP, with cytokine concentrations independently being 1-20 ng/mL.

In some more preferred embodiments of the present disclosure, the cytokines include 1-5 cytokines selected from bFGF, IGF, HGF, TGF-β1, TGF-β2, TGF-β3, and BMP, with all cytokine concentrations being 5-15 ng/mL.

In some more preferred embodiments of the present disclosure, the cytokines include bFGF, VEGF, TGF-β1, and TGF-β2, with cytokine concentrations independently being 5-15 ng/mL.

In some more preferred embodiments of the present disclosure, the cytokines are bFGF, VEGF, TGF-β1, and TGF-β2, with cytokine concentrations independently being 5-15 ng/mL.

In some embodiments of the present disclosure, the vitamins include vitamin B and vitamin C.

In some preferred embodiments of the present disclosure, the vitamins include vitamin B and vitamin C, with concentrations independently being 30-100 mg/mL.

In some embodiments of the present disclosure, the amino acids include glycine, alanine, glutamic acid, and proline.

In some preferred embodiments of the present disclosure, the amino acids include glycine, alanine, glutamic acid, and proline, with concentrations independently being 20-60 mg/mL.

In some embodiments of the present disclosure, the serum substitute is Human Platelet Lysate (HPL).

In the technical scheme of the present disclosure, by using optimized and screened cytokines and their added concentrations and combinations, a preferred secretion-promoting culture medium is obtained. The secretion-promoting culture medium can effectively promote the interaction between cells and secretion of structural and supportive proteins, facilitate rapid formation of vascular tissue, and enhance the elasticity and mechanical strength of the vascular tissue, thereby enabling the blood vessels prepared by the technical scheme of the present disclosure to have good long-term patency.

In the technical scheme of the present disclosure, by optimizing the secretion-promoting culture medium and adding appropriate concentrations and types of vitamins and amino acids, cell growth and secretion of extracellular matrix components such as structural and supportive proteins by cells are promoted, and a synergistic effect is exerted with cytokines to promote maturation of vascular tissue and enhance the elasticity and mechanical strength of the artificial blood vessel.

In the technical scheme of the present disclosure, the growth rate of vascular cells can also be effectively improved and the preparation time of the artificial blood vessel can be shortened by using the serum substitute HPL to replace serum.

In some embodiments of the present disclosure, the seeding of the vascular fibroblasts in step S2 is performed by dynamic seeding, with a seeding time of 1 to 4 days.

In some preferred embodiments of the present disclosure, the seeding of the vascular fibroblasts seeding in step S2 is performed by dynamic seeding, with a seeding time of 2 to 4 days.

In a second aspect, the present disclosure provides a tissue-engineered artificial blood vessel prepared by the preparation method of the first aspect of the present disclosure.

Since the tissue-engineered artificial blood vessel according to the second aspect of the present disclosure employs the entire technical scheme of the preparation method described in the above embodiments, it possesses at least all the beneficial effects brought by the technical scheme of the above embodiments.

In some embodiments of the present disclosure, a scaffold material accounts for 0%-10% by mass in the artificial blood vessel.

In some preferred embodiments of the present disclosure, a scaffold material accounts for 0%-6% by mass in the artificial blood vessel.

In some embodiments of the present disclosure, the artificial blood vessel has an inner diameter of 1.5-15 mm.

In some preferred embodiments of the present disclosure, the artificial blood vessel has an inner diameter of 2-10 mm.

In some more preferred embodiments of the present disclosure, the artificial blood vessel has an inner diameter of 2-6 mm.

In some embodiments of the present disclosure, the artificial blood vessel has a wall thickness of 0.1-2 mm.

In some preferred embodiments of the present disclosure, the artificial blood vessel has a wall thickness of 0.2-1.5 mm.

In some more preferred embodiments of the present disclosure, the artificial blood vessel has a wall thickness of 0.2-1 mm.

In a third aspect, the present disclosure provides use of the preparation method of the first aspect and the tissue-engineered artificial blood vessel of the second aspect in A1)-A5):
A1) a vascular product and preparation thereof;
A2) a product for treating vascular diseases and preparation thereof;
A3) an artificial intestine, an artificial esophagus, and other tubular tissue product and preparation thereof;
A4) a blood circulation simulation device in vitro and preparation thereof; and
A5) a vascular implant device in vivo and preparation thereof.

Other features and advantages of the present disclosure will be set forth in the description which follows, and in part will be apparent from the description, or may be learned by practice of the present disclosure.

### BRIEF DESCRIPTION OF DRAWINGS

The present disclosure will be further described in conjunction with the accompanying drawings and embodiments, in which:
FIG. 1 is a photographic showing the appearance of a tissue-engineered artificial blood vessel of Example 1 of the present disclosure;
FIG. 2 shows hematoxylin-eosin (HE) staining results of the tissue-engineered artificial blood vessel of Example 1 of the present disclosure; and
FIG. 3 is a photographic showing the appearance of a tissue-engineered artificial blood vessel of Comparative Example 2 of the present disclosure.

### DETAILED DESCRIPTION

The conception and technical effects produced of the present disclosure will be described clearly and completely below with reference to the embodiments, so as to facilitate a full understanding of the objectives, features and effects of the present disclosure. Obviously, the described embodiments are merely part of the embodiments of the present disclosure rather than all of them. Any other embodiment obtained by those having ordinary skills in the art based on the embodiments of the present disclosure without creative effort shall fall within the scope of protection of the present disclosure.

If specific conditions are not indicated in the embodiments, the conventional conditions or the conditions recommended by the manufacturer shall be followed. The reagents or instruments used without indicating the manufacturer are all conventional products commercially available.

The endothelial cell culture medium used in the examples was purchased from Lonza, Cat. No. CC-3156; the smooth muscle cell culture medium used was purchased from Lonza, Cat. No. CC-3182; and the fibroblast culture medium used was purchased from Sciencell, Cat. No. 2301.

The biodegradable polymer tubular cellular scaffold used in the examples was a high-porosity tubular material processed from polyglycolic acid polymer, purchased from Suzhou Qianyun Instrument Technology Co., Ltd., product model: BIOFELT/PGA. The product purchase website is: http://www.genintech.com/content/?164.html.

The vascular endothelial cells, smooth muscle cells, and fibroblasts used in the examples were purchased from Wuhan Procell Life Science & Technology Co., Ltd. The product purchase website is: https://www.procell.com.cn/xbzy/ydxb/.

### Example 1

This example provides a preparation method for a small-caliber artificial blood vessel with an inner diameter of 2 mm. The specific steps were as follows.
(1) Endothelial cells and smooth muscle cells isolated from human umbilical arteries in vitro were prepared. An endothelial cell culture medium and a smooth muscle cell culture medium were mixed at a volume ratio of 1:4 to obtain a mixed culture medium 1, which had a serum mass concentration of 20% and was supplemented with 1% double antibiotics (penicillin and streptomycin). The endothelial cells and smooth muscle cells were resuspended in the mixed culture medium 1 to achieve a density ratio of approximately 1:10, thereby obtaining a cell suspension. The preparation of the cell suspension and all culture media was performed in a sterile isolator.
(2) A biodegradable polymer tubular cellular scaffold having a wall thickness of 0.8 mm and an inner diameter of 2 mm was prepared and placed in a vascular bioreactor. The cell suspension from step (1) was introduced into the cellular scaffold of the vascular bioreactor through aseptic connection to a fluidic circuit perfusion system to seed cells on an inner layer of the cellular scaffold, where the endothelial cells had a seeding density of 200,000 cells/cm , the smooth muscle cells had a seeding density of 2,000,000 cells/cm, and the culture was performed in a tissue culture incubator at 37°C with a carbon dioxide concentration of 5% and a humidity above 90%.
(3) After 10 days of culture, the endothelial cells and smooth muscle cells proliferated rapidly and formed an endothelial cell layer and a smooth muscle cell layer. The mixed culture medium 1 was replaced with a secretion-promoting culture medium 1, which was prepared by reducing a serum concentration to 10% based on the mixed culture medium 1, and supplementing with growth factors VEGF, TGF-β1, and TGF-β2 at a final concentration of 10 ng/mL each, glycine, alanine, glutamic acid, and proline at a final concentration of 40 mg/mL each, and vitamin B and vitamin C at a final concentration of 70 mg/mL each.
(4) The culture was continued for another 10 days. Meanwhile, an external peristaltic pump was used to control flow rate changes to apply a perfusion pulsatile pressure of 1-300 mmHg to the pipeline and scaffold via radial pulsatile stimulation of 1%-10% amplitude. The perfusion pulsatile pressure was increased proportionally by 5 mmHg daily starting from an initial pressure of 1 mmHg. Meanwhile, a fibroblast suspension resuspended in a fibroblast culture medium was introduced into an outer layer culture medium of the vascular scaffold for seeding. The fibroblasts were also isolated from human umbilical arteries in vitro and had a seeding density of 1,000,000 cells/cm. A circulation time for the fibroblast suspension introduced into the outer layer culture medium was 3 days.
(5) A mixed culture medium 2 was prepared, where a volume ratio the endothelial cell culture medium: the smooth muscle cell culture medium: the fibroblast culture medium was 1:6:3, with a serum mass concentration of 10%. Both the inner and outer layer culture media were replaced with the mixed culture medium 2 for three-cell co-culture for 10 days, and a fibroblast layer with a thickness of 0.05-0.1 mm was formed on the outer layer of the scaffold. The mixed culture medium 2 was replaced with a secretion-promoting culture medium 2, which was prepared by reducing the serum concentration to 5% based on the secretion-promoting culture medium 1, and supplementing with growth factor bFGF at a concentration of 10 ng/mL. Meanwhile, the perfusion pulsatile pressure was gradually increased by 10-15 mmHg daily. The cellular scaffold gradually degraded, and the degraded portion were replaced by cells and extracellular matrix components such as structural and supportive proteins secreted by the cells.
(6) The culture was continued for 2 weeks in the secretion-promoting culture medium 2 to form a tubular tissue rich in cells and extracellular matrix components such as structural and supportive proteins with small amounts of scaffold material. An in-situ decellularization treatment was performed to obtain the tissue-engineered artificial blood vessel.

The specific steps of the in-situ decellularization treatment were as follows: after completion of the culture, replacing the culture medium with a decellularization solution containing SDS at a mass concentration of 1%, TritonX-100 at a mass concentration of 1%, and CHAPS at a molar concentration of 8 mM, followed by stirring at 25 rpm at 37°C or by perfusing with the decellularization solution for 48 h to complete the decellularization treatment.

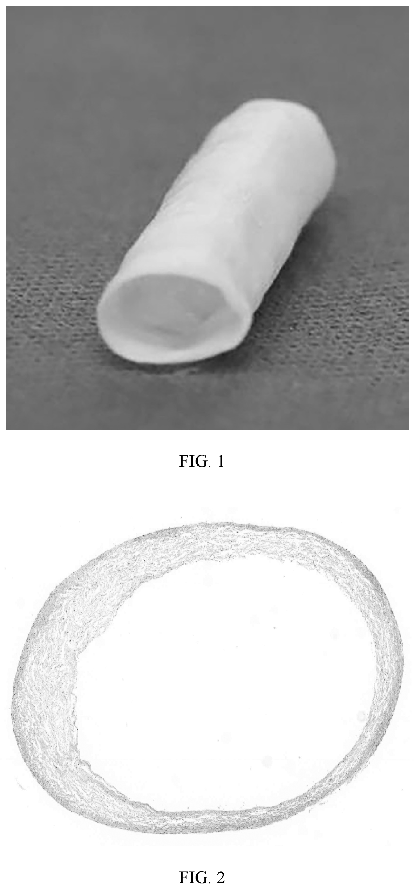

The preparation time of the artificial blood vessel in this example was approximately 7 weeks. The tissue-engineered artificial blood vessel was measured to have an inner diameter of 2 mm and a wall thickness of 0.3 mm. A residual rate of the polymer material in the tested artificial blood vessel was measured to be 5.3%. A photograph of the tissue-engineered artificial blood vessel prepared in this example was taken, as shown in FIG. 1. Histological sections of the tissue-engineered artificial blood vessel prepared in this example were stained with HE, as shown in FIG. 2. The predominant red staining in the vessel wall indicates that the tissue-engineered artificial blood vessel prepared in this example mainly includes extracellular matrix composed of structural and supportive proteins.

### Example 2

This example provides a preparation method for a medium-large caliber artificial blood vessel with an inner diameter of 6 mm. The specific steps were as follows.
(1) Endothelial cells and smooth muscle cells isolated from human umbilical arteries in vitro were prepared. An endothelial cell culture medium and a smooth muscle cell culture medium were mixed at a volume ratio of 1:4 to obtain a mixed culture medium 1, which had a serum mass concentration of 20% and was supplemented with 1% double antibiotics (penicillin and streptomycin). The endothelial cells and smooth muscle cells were resuspended in the mixed culture medium 1 to achieve a density ratio of approximately 1:6, thereby obtaining a cell suspension. The preparation of the cell suspension and all culture media was performed in a sterile isolator.
(2) A biodegradable polymer tubular cellular scaffold having a wall thickness of 1 mm and an inner diameter of 6 mm was prepared and placed in a vascular bioreactor. The cell suspension from step (1) was introduced into the cellular scaffold of the vascular bioreactor through aseptic connection to a fluidic circuit perfusion system to seed cells on an inner layer of the cellular scaffold, where the endothelial cells had a seeding density of 800,000 cells/cm , the smooth muscle cells had a seeding density of 5,000,000 cells/cm, and the culture was performed in a tissue culture incubator at 37°C with a carbon dioxide concentration of 5% and a humidity above 90%.
(3) After 8 days of culture, the endothelial cells and smooth muscle cells proliferated rapidly and formed an endothelial cell layer and a smooth muscle cell layer. The mixed culture medium 1 was replaced with a secretion-promoting culture medium 1, which was prepared by reducing a serum concentration to 10% based on the mixed culture medium 1, and supplementing with growth factors VEGF, TGF-β1, and TGF-β2 at a concentration of 10 ng/mL each, glycine, alanine, glutamic acid, and proline at a final concentration of 40 mg/mL each, and vitamin B and vitamin C at a final concentration of 70 mg/mL each.
(4) The culture was continued for another 10 days. Meanwhile, an external peristaltic pump was used to control flow rate changes to apply a perfusion pulsatile pressure of 1-300 mmHg to the pipeline and scaffold via radial pulsatile stimulation of 1%-10% amplitude. The perfusion pulsatile pressure was increased proportionally by 5 mmHg daily starting from an initial pressure of 1 mmHg. Meanwhile, a fibroblast suspension resuspended in a fibroblast culture medium was introduced into an outer layer culture medium of the vascular scaffold for seeding. The fibroblasts were also isolated from human umbilical artery and vein in vitro and had a seeding density of 3,000,000 cells/cm. A circulation time for the fibroblast suspension introduced into the outer layer culture medium was 3 days.
(5) A mixed culture medium 2 was prepared, where a volume ratio the endothelial cell culture medium: the smooth muscle cell culture medium: the fibroblast culture medium was 1:6:3, with a serum mass concentration of 10%. Both the inner and outer layer culture media were replaced with the mixed culture medium 2 for three-cell co-culture for 10 days, and a fibroblast layer with a thickness of 0.8-1 mm was formed on the outer layer of the scaffold. The mixed culture medium 2 was replaced with a secretion-promoting culture medium 2, which was prepared by reducing the serum concentration to 5% based on the secretion-promoting culture medium 1, and supplementing with growth factor bFGF at a concentration of 10 ng/mL. Meanwhile, the perfusion pulsatile pressure was gradually increased by 10-15 mmHg daily. After 1 week of culture, the cellular scaffold gradually degraded, and the degraded portion were replaced by cells and extracellular matrix components such as structural and supportive proteins secreted by the cells.
(6) The culture was continued for 2 weeks in the secretion-promoting culture medium 2 system to form a tubular tissue rich in cells and extracellular matrix components such as structural and supportive proteins with small amounts of scaffold material. An in-situ decellularization treatment was performed to obtain the tissue-engineered artificial blood vessel.

The preparation time of the artificial blood vessel in this example was less than 7 weeks. The tissue-engineered artificial blood vessel was measured to have an inner diameter of 6 mm and a wall thickness of 1 mm.

### Example 3

This example provides a preparation method for a small-caliber artificial blood vessel with an inner diameter of 2 mm.

The difference between this example and Example 1 lies in that: the serum in all the culture media of Example 1 was replaced with the serum substitute HPL, where the serum mass concentration of 20% in the mixed culture medium 1 was replaced with 15% HPL concentration, the serum concentration of 10% in the secretion-promoting culture medium 1 was replaced with 10% HPL concentration, the serum concentration of 10% in the mixed culture medium 2 was replaced with 10% HPL concentration, and the serum concentration of 5% in the secretion-promoting culture medium 2 was replaced with 5% HPL concentration; the culture in step (3) was performed for 6 days instead of 10 days, the culture in step (4) was performed for 8 days instead of 10 days, and the circulation time in step (4) was 2 days instead of 2 to 4 days, with the remaining culture times unchanged. As a result, the overall culture time was shortened by approximately 1 week as compared to Example 1.

### Example 4

This example provides a preparation method for a small-caliber artificial blood vessel with an inner diameter of 2 mm.

The differences between this example and Example 1 lie in that: the seeding concentrations of all the three types of cells in Example 1 were increased, with the seeding density of the endothelial cells changed from 200,000 cells/cm to 400,000 cells/cm, the seeding density of the smooth muscle cells changed from 2,000,000 cells/cm to 4,000,000 cells/cm, and the seeding density of the fibroblasts changed from 1,000,000 cells/cm to 2,000,000 cells/cm; the culture in step (3) was performed for 6 days instead of 10 days , the culture in step (4) was performed for 8 days instead of 10 days, and the circulation time in step (4) was 2 days instead of 2 to 4 days, with the remaining culture times unchanged. As a result, the overall culture time was shortened by approximately 1 week as compared to Example 1.

### Example 5

This example provides a preparation method for a small-caliber artificial blood vessel with an inner diameter of 2 mm.

The difference between this example and Example 1 lies in that: the volume ratio of the endothelial cell medium to the smooth muscle cell medium in step (1) was changed from 1:4 to 1:10, without changing the density ratio and seeding density of the two types of cells; and the volume ratio of the endothelial cell culture medium, the smooth muscle cell culture medium, and the fibroblast culture medium in step (5) was changed from 1:6:3 to 1:10:5.

The artificial blood vessel prepared in this example exhibited somewhat reduced growth rates of individual cell layers and decreased interaction effects between different cells compared to Example 1. Under the same culture time, the wall thickness of the artificial blood vessel of this example was reduced compared to Example 1.

### Comparative Example 1

This comparative example provides a preparation method for a small-caliber artificial blood vessel with an inner diameter of 2 mm.

The differences between this comparative example and Example 1 lie in that: no secretion-promoting culture medium was not used during the culture process; the secretion-promoting culture medium 1 was replaced with the mixed culture medium 1, and the secretion-promoting culture medium 2 was replaced with the mixed culture medium 2, with all other culture parameters and intervention parameters remaining exactly the same as in Example 1.

The final artificial blood vessel product obtained in this comparative example still formed a relatively complete white tubular tissue, but the formed tissue lacked sufficient elasticity and mechanical properties, and failed to secrete adequate amounts of extracellular matrix components such as structural and supportive proteins.

### Comparative Example 2

This comparative example provides a preparation method for a small-caliber artificial blood vessel with an inner diameter of 2 mm.

The difference between this comparative example and Example 1 only lies in that: no pulsatile pressure stimulation was given during the culture process, and the culture was performed under static conditions throughout.

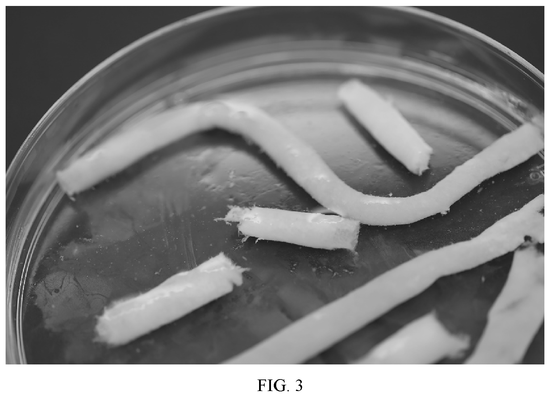

The artificial blood vessel prepared in this comparative example could form intact vascular tissue after decellularization treatment. However, the tissue exhibited insufficient secretion of extracellular matrix components such as structural and supportive proteins, and lacked sufficient elasticity and mechanical strength. As shown in the photograph of the tissue-engineered artificial blood vessel in FIG. 3, the artificial blood vessel of this comparative example is prone to fracture, with poor elasticity and mechanical properties.

### Comparative Example 3

This comparative example provides a preparation method for a small-caliber artificial blood vessel with an inner diameter of 2 mm.

The differences between this comparative example and Example 1 lie in that: the culture in step (3) was performed for 6 days instead of 10 days, the culture in step (4) was performed for 8 days instead of 10 days, and the circulation time in step (4) was 2 days instead of 2 to 4 days, with the remaining culture times unchanged.

Due to the shortened culture times in multiple periods compared to Example 1, the proliferation of cells in each layer and secretion of extracellular matrix in the artificial blood vessel prepared in this comparative example were insufficient, resulting in an artificial blood vessel having a thinner wall and exhibiting inferior elasticity and mechanical strength.

### Test Example

Physical property tests were conducted on the artificial blood vessels prepared in Examples 1-4 and Comparative Examples 1-2 to characterize the elasticity and mechanical strength of the artificial blood vessels. The specific test methods are as follows. According to the methods specified in the industry standard YY/T0500-2021 *"Cardiovascular Implants"* the axial tensile strength, circumferential tensile strength, pressurized burst strength, and suture tensile strength of the artificial blood vessels were tested:
1. Axial Tensile Strength: it was measured on tubular vascular grafts in their tubular configuration. In the test, both ends of the blood vessel were fixed in grips of a material testing machine with a grip spacing of 50 mm. Care was taken to ensure that the sample was not stretched, twisted, or damaged by the grips, and was maintained in a natural state as much as possible. The sample was then stretched at a constant rate of 150 mm/min until fracture, and a load value at a fracture point was recorded as the axial tensile strength.
2. Circumferential Tensile Strength: it was measured on the tubular vascular grafts in their tubular configuration along a circumferential direction. A test sample with a length of 40 mm was cut from the sample, recorded as a sample length (L), in mm. The sample was placed on two pins of the grips of a universal testing machine, ensuring that the sample was neither stretched nor twisted and maintained in a natural state. Then, the sample was stretched at a rate of 150 mm/min until a fracture point was reached. The maximum load (Tmax) was recorded. The circumferential tensile strength was calculated by dividing the maximum load (Tmax) of each sample by its original sample length, using the formula: Circumferential Tensile Strength = Tmax/2L.
3. Pressurized Burst Strength: the vascular tissue was connected to a pressurized perfusion device. Fluid was pumped into the blood vessel at 10 kPa/s to 25 kPa/s, allowing the pressure to increase steadily while being recorded in real-time. The pressure at which the blood vessel ruptured was recorded as the pressurized burst strength.
4. Suture Tensile Strength: it was measured as a force required to pull a suture out of the wall of a tubular vascular graft. A sample approximately 20 mm in length was cut along an axial direction. A 6-0 nylon suture (with a diameter of 0.16 mm) was inserted through one layer of the vessel wall at 2 mm from one end of the straightened sample to form a half-loop. The suture was pulled at a rate of 150 mm/min, and a force required to withdraw the suture from the vessel wall or cause damage to the wall was recorded.

The test results are shown in Table 1.

**Table 1: Physical Property Data of Artificial Blood Vessels Prepared in Examples 1-4 and Comparative Examples 1-2**

| Test Item | Example 1 | Example 2 | Example 3 | Example 4 | Comparative Example 1 | Comparative Example 2 |
|---|---|---|---|---|---|---|
| Axial Tensile Strength (MPa) | 2.33 | 2.11 | 2.03 | 2.27 | 0.35 | 0.43 |
| Circumferential Tensile Strength (N/mm) | 2.01 | 2.10 | 2.04 | 2.06 | 0.33 | 0.41 |
| Pressurized Burst Strength (kPa) | 266.43 | 287.56 | 288.11 | 297.23 | 30.42 | 43.65 |
| Suture Tensile Strength (N) | 1.88 | 2.04 | 1.98 | 2.06 | 0.27 | 0.41 |

As can be seen from the above test results, the artificial blood vessels prepared in Examples 1-4 of the present disclosure have significantly higher axial tensile strength, circumferential tensile strength, pressurized burst strength, and suture retention strength compared to those prepared in Comparative Examples 1-2. The values are basically an order of magnitude higher than those of the comparative examples, indicating that the preparation method for the artificial blood vessel of the present disclosure has obvious advantages in promoting cell secretion of extracellular matrix components such as structural and supportive proteins, and in forming tissues with higher elasticity and strength, thereby giving the preparation method for an artificial blood vessel and the artificial blood vessel prepared of the present disclosure good application value.

In summary, the preparation method for a tissue-engineered artificial blood vessel of the present disclosure can be used to prepare small-caliber artificial blood vessels with an inner diameter of less than 6 mm. During the culture and preparation process, it promotes substantial secretion of extracellular matrix components by cells, enabling the obtained tissue-engineered artificial blood vessel to have high elasticity, good mechanical properties and biocompatibility. The artificial blood vessel prepared by the present disclosure has high contents of structural and supportive proteins, enabling the vessel wall to maintain good elasticity and mechanical properties, thereby exhibiting excellent long-term patency in practical use. In addition, the preparation method for an artificial blood vessel of the present disclosure employs simultaneous culture of three types of cells, shortening the culture time, reducing costs, and mitigating the risk of contamination. The tissue-engineered artificial blood vessel and preparation method of the present disclosure have good application prospects in vascular products, products for treating vascular diseases, and artificial intestines, artificial esophagus, and other tubular tissue products.

The embodiments of the present disclosure are described in detail above, but the present disclosure is not limited to the above embodiments. Within the scope of knowledge possessed by those having ordinary skills in the art, various modifications can be made without departing from the purpose of the present disclosure. Furthermore, the embodiments of the present disclosure and features in the embodiments may be combined with each other if not in conflict.

## Claims

1. A preparation method for a tissue-engineered artificial blood vessel, comprising the following steps:
S1: seeding vascular endothelial cells and smooth muscle cells on an inner layer of a tubular cellular scaffold, and performing mixed culture for 7 to 13 days;
S2: changing to a secretion-promoting culture medium, simultaneously applying perfusion pulsatile pressure within the tubular cellular scaffold, continuing culture for 8-13 days, then seeding vascular fibroblasts on an outer layer of the tubular cellular scaffold for culture, and changing to a mixed culture medium for co-culture for 8 to 13 days; and
S3: replacing the mixed culture medium with the secretion-promoting culture medium, co-culturing for 10 to 16 days, followed by in-situ decellularization treatment to obtain the tissue-engineered artificial blood vessel;
wherein the secretion-promoting culture medium comprises cytokines, amino acids, vitamins, and serum or serum substitute.

2. The preparation method of claim 1, wherein the endothelial cells, the smooth muscle cells, and the fibroblasts are derived from an aorta and an umbilical artery and vein;
preferably, the endothelial cells have a seeding density of 100,000-1,500,000 cells/cm, the smooth muscle cells have a seeding density of 1,000,000-7,000,000 cells/cm, and the fibroblasts have a seeding density of 500,000-7,000,000 cells/cm.

3. The preparation method of claim 1, wherein the tubular cellular scaffold is made of a biodegradable polymer material;
the tubular cellular scaffold has a wall thickness of 0.1-2.5 mm;
preferably, a component of the tubular cellular scaffold is at least one selected from the group consisting of polylactic acid, polyglycolic acid, polycaprolactone, poly(lactic-co-glycolic acid), polyhydroxyalkanoates, and collagen.

4. The preparation method of claim 1, wherein a step of applying perfusion pulsatile pressure in step S2 comprises initially applying 1-10 mmHg, then increasing by 3-20 mmHg daily.

5. The preparation method of claim 1, wherein the culture medium for mixed culture in step S1 is a mixture of a vascular endothelial cell culture medium and a smooth muscle cell culture medium in a volume ratio of 1: 2-10, and the mixed culture medium in step S2 is a mixture of a vascular endothelial cell culture medium, a smooth muscle cell culture medium, and a fibroblast culture medium in a volume ratio of 1: 4-10: 2-5;
preferably, the culture medium for mixed culture in step S1 contains serum or serum substitute HPL at a mass concentration of 10%-25%;
preferably, the mixed culture medium in step S2 contains serum or serum substitute HPL at a mass concentration of 5%-15%.

6. The preparation method of claim 1, wherein the secretion-promoting culture medium in step S2 comprises cytokines, amino acids, vitamins, and 8%-12% serum or serum substitute;
wherein the cytokines comprise at least one of VEGF, PDGF, IGF, HGF, TGF-β1, TGF-β2, TGF-β3, and BMP;
the amino acids comprise glycine, alanine, glutamic acid, and proline; and
the vitamins comprise vitamin B and vitamin C.

7. The preparation method of claim 1, wherein the secretion-promoting culture medium in step S3 comprises cytokines, amino acids, vitamins, and 3%-7% serum or serum substitute;
wherein the cytokines comprise at least one of bFGF, IGF, HGF, TGF-β1, TGF-β2, TGF-β3, and BMP;
the amino acids comprise glycine, alanine, glutamic acid, and proline; and
the vitamins comprise vitamin B and vitamin C.

8. A tissue-engineered artificial blood vessel prepared by the preparation method of any one of claims 1 to 7.

9. The tissue-engineered artificial blood vessel of claim 8, wherein a scaffold material accounts for 0-10% by mass in the artificial blood vessel;
preferably, the artificial blood vessel has an inner diameter of 1.5-15 mm; and
more preferably, the artificial blood vessel has a wall thickness of 0.1-2 mm.

10. Use of the preparation method of any one of claims 1 to 7 or the artificial blood vessel of claim 9 in A1)-A5):
A1) a vascular product and preparation thereof;
A2) a product for treating vascular diseases and preparation thereof;
A3) an artificial intestine, an artificial esophagus, and other tubular tissue products and preparation thereof;
A4) a blood circulation simulation device in vitro and preparation thereof; and
A5) a vascular implant device in vivo and preparation thereof.
